# EUROPEAN PATENT SPECIFICATION

(11) **EP 0 541 215 B1**
(45) Date of publication and mention of the grant of the patent: **16.10.1996**
(21) Application number: 92307499.1
(22) Date of filing: 14.08.1992
(51) Int. Cl.: A61F 2/24

(54) **Heart valve prosthesis**
Herzklappenprothese
Prothèse de valvule cardiaque

(30) Priority: 31.10.1991 US 786253
(43) Date of publication of application: 12.05.1993
(73) Proprietor: ATS MEDICAL, INC., Plymouth, Minnesota 55447 (US)
(72) Inventor: Hanson, Donald W., Chanhassen, Minnesota 55317 (US); Kramp, Richard W., Arden Hills, Minnesota 55112 (US); Villafana, Manuel A., Minneapolis, Minnesota 55447 (US)
(74) Representative: MacGregor, Gordon

(56) References cited:
- EP-A- 0 176 337
- EP-A- 0 207 594
- WO-A-90/08519
- WO-A-91/01698
- WO-A-92/02197

## Description

### BACKGROUND OF THE INVENTION

The present invention relates generally to an improved heart valve prosthesis, and more particularly to such a prosthesis device which may be implanted to replace the defective natural heart valve. The improved prosthesis device of the present invention employs a pair of occluder means, each in the form of a flat plate, both functioning hemodynamically through periodic opening and closing motion which is created through normal pumping action of the heart.

Prosthetic heart valves function essentially as check valves. Blood flow which occurs as a result of the natural pumping action of the heart causes periodic opening of the occluder means, with the system pressure closing the occluder means during periods of diastole when in the aortic position or during periods of systole when in the atrio-ventricular position.

A variety of prosthetic heart valves have been proposed and utilized in the past. Certain of these prosthetic devices have employed a caged ball arrangement which also function and control blood flow in response to the normal pumping action of the heart. Other heart valve prostheses have employed occluders in the form of either a round disc or a pair of semi-circular and semi-elliptical plates. The latter are normally referred to as bi-leaflet valves. While various materials of construction have been employed in the past, the more recently utilized heart valve prostheses have been fabricated essentially from pyrolytic carbon. The improved prosthetic heart valve of the present invention is a bi-leaflet valve fabricated from pyrolytic carbon.

Bi-leaflet heart valves normally employ pivot means to appropriately guide and otherwise control the motion of the leaflets through their transition from open disposition to closed disposition. In addition, means have been provided to control or limit the extent of motion to which the leaflets are subjected during opening and closing, thereby providing an arrangement wherein the motion of the individual leaflets is carefully guided, controlled, limited, and maintained.

The present invention set out in Claim 1, and is a modification of that certain heart valve prosthesis disclosed and claimed in EP-A-0176337 (corresponding to US Patents Nos 4,692,165, Bokros and 4,822,353, Bokros). The precharacterising part of Claim 1 is based on EP-A-0176337, and the distinguishing features of the present invention are set out in the characterising part of Claim 1.

The structure of the present invention utilizes modified forms of pivot means and occluder stop means, all of which combine to provide modified forms of flow characteristics through the valve prosthesis. The structural configuration involved in the device of the present invention is designed to improve the flow dynamics and reduce areas of statis, and, significantly reduce if not eliminate the formation of eddies in the flow pattern through the valve.

It is known that blood components including those cells normally found in human blood are extremely fragile and delicate, and that these cells can be damaged and/or destroyed when subjected to unusual mechanical forces. Thus, care must be taken in order to control the nature of the forces created due to the occurrence of relative motion between the leaflets and the annular body. For example, reduction of the occurrences of rubbing contact between stationary and moving surfaces is of importance when such contact is likely to cause mechanical damage to the components or cells present in blood. The design and configuration of the heart valve prosthesis of the present invention is such that care has been taken to reduce the creation of zones or areas where blood passing through the device is exposed to substantial mechanical forces. In addition to damage due to mechanical forces, it has been found that constituents of human blood are subject to damage whenever any quantity of blood is retained or held in an area or zone of stasis or stagnation within a prosthesis. The design and configuration of the heart valve prosthesis of the present invention is such that areas of stasis are reduced, if not entirely eliminated.

The heart valve prosthesis of the present invention comprises a generally annular body member having an interior surface defining a central passageway for blood flow therethrough. The annular body member is provided with means for supporting a pair of pivotally movable leaflets or occluders within the annular body for alternately and periodically opening and closing, thereby allowing a unidirectional flow of blood through the passageway. The leaflets comprising the occluder means are provided with aligned, inwardly extending notches or cut-out zones providing concavities at opposed locations along the periphery. These concavities are arranged to mate with oppositely disposed pairs of inwardly projecting occluder supports or pivot areas in the form of spherical segments which extend convexly inwardly from opposed chordal locations within the annular body member. In this manner, the spherical segments form pivot areas or fulcrum points which engage and are received within the opposed aligned concavities. In this connection, the axes of the pivot areas are arranged along and generally coincidental with the axis extending between the concavities formed within individual leaflets. Stop means are provided which extend inwardly of the interior surface of the annular body member and cooperate with the pivot areas and the leaflets to control and otherwise limit the extent of pivotal motion of each leaflet during its opening and closing. The stop means are arranged to reduce the magnitude of forces created on the blood retained along and between the surfaces of the stop means and the individual leaflets.

In normal operation, the leaflets comprising the occluder means pivot about an axis extending between individual ones of opposed pairs of fulcrum points or pivot areas. While the individual abutment surfaces of the stop means provide relatively elongated zones or areas of contact, additional contact is achieved between the mating surfaces of the elliptical edges of the leaflets and the interior surface of the annular body. Additionally, areas of mutual contact exist during closure between the straight-line end zones of the individual leaflets. This utilization of areas of contact assists in reduction of mechanical forces to which the blood is exposed during normal operation of the prosthesis.

Preferably, the occluder means undergo a rotational skirt of motion between open and closed dispositions of about 60 degrees.

Therefore, it is a primary object of the present invention to provide an improved heart valve prosthesis means which is designed to provide improved performance while responding hemodynamically to the normal pumping action of the heart.

It is a further object of the present invention to provide an improved heart valve prosthesis which is designed to reduce exposure of blood and its constituents to substantial mechanical forces and stresses, thereby reducing the likelihood of damage to constituents and cells found within human blood.

It is yet a further object of the present invention to provide an improved heart valve prosthesis with mechanical design features and characteristics which reduce the occurrence of a concentration of mechanical stresses at given locations in the device.

It is yet a further object of the present invention to provide an improved heart valve prosthesis which is designed to enhance the uniformity of flow through the device so as to substantially reduce the creation of zones of stasis or stagnation within the device and its environs.

Other and further objects of the present invention will become apparent to those skilled in the art upon a study of the following specification, appended claims, and accompanying drawings.

### IN THE DRAWINGS

Figure 1 is a perspective view of the heart valve prosthesis of the present invention, with the valve being shown from the inflow end, and shown with the leaflets in open position;
Figure 2 is a vertical sectional view taken along the line and in the direction of the arrows 2-2 of Figure 1;
Figures 3 and 4 are views similar to Figure 2, with the leaflets being shown in the closed position in Figure 3, and with the leaflets having been removed in the view of Figure 4;
Figure 5 is a vertical sectional view taken along the line and in the direction of the arrows 5-5 of Figure 2;
Figure 6 is a vertical sectional view taken along the line and in the direction of the arrows 6-6 of Figure 4, and showing one interior end of the heart valve prosthesis;
Figure 7 is an inflow plan view of the heart valve prosthesis, with the leaflets being shown in the open position; and
Figure 8 is a fragmentary vertical sectional view taken through a portion of the annular body, and illustrating the detail of the sewing cuff assembly secured to the outer circumferential surface of the valve assembly, with Figure 8 being shown on a slightly enlarged scale.

### DESCRIPTION OF THE PREFERRED EMBODIMENT

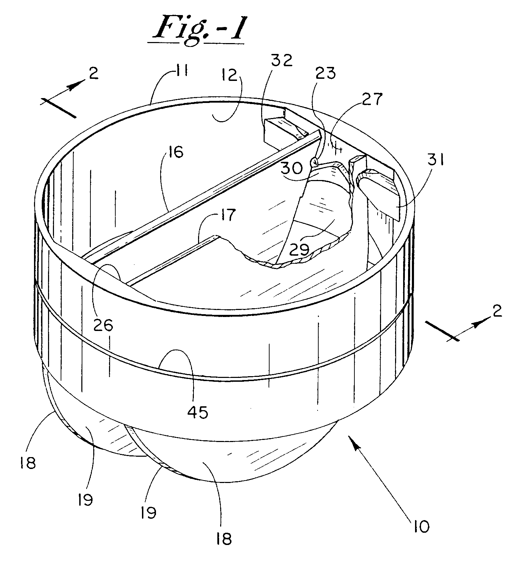

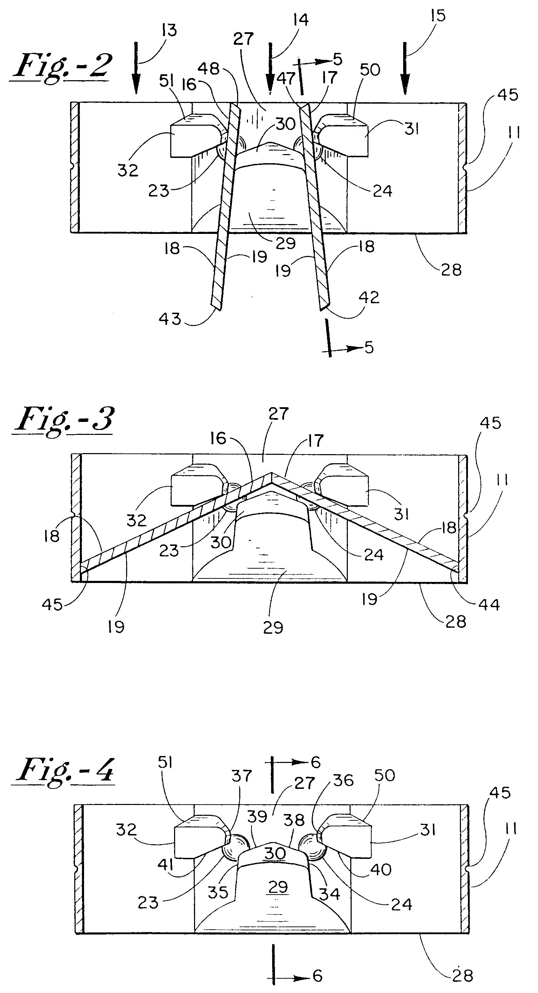

In accordance with the preferred embodiment of the present invention, and with particular attention being directed to Figures 1 and 2 of the drawings, the heart valve prosthesis generally designated 10 comprises a generally annular body member 11 having an interior surface 12 defining a central passageway for blood flow therethrough. With attention being directed to Figure 2, the directional arrows 13, 14, and 15 illustrate the typical flow pattern for blood entering the inflow end and passing through the heart valve prosthesis 10, with portions of the flow occurring between the occluder members and the surface of the annular body member, and with further portions of the blood flow passing between the surfaces of the individual leaflets. Occluder means including leaflets 16 and 17 are provided, with the leaflets having upstream directed major surfaces 18-18, and downstream directed major surfaces 19-19. As indicated, the occluder means respond hemodynamically to the natural pumping action of the heart, so as to alternately open and close so as to permit flow of blood through the passageway upon occurrence of an increase in pressure on the inflow side so as to cause a positive pressure differential relative to the outflow side of the device. Closure occurs as the relative pressures become positive with respect to the outflow side.

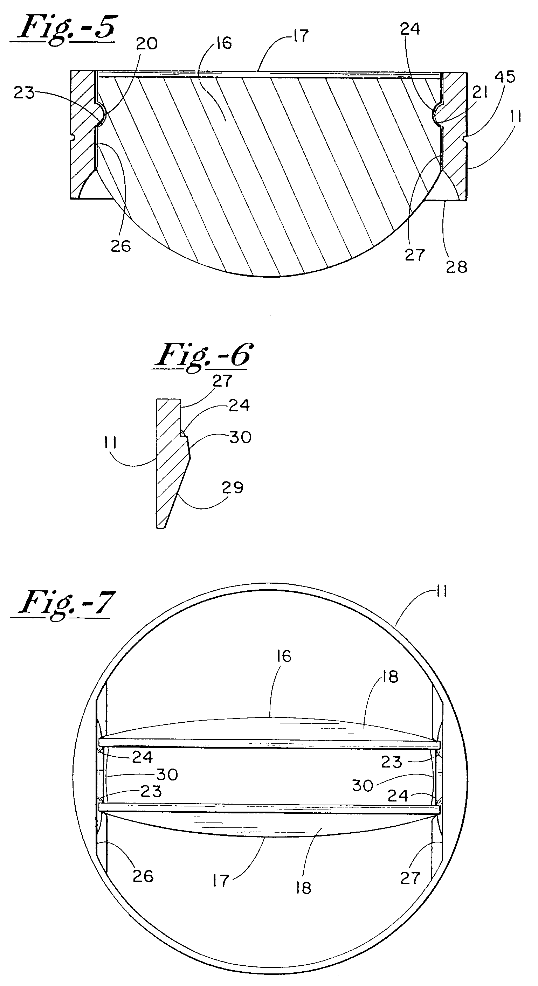

With attention now being directed to Figure 5 of the drawings, each leaflet 16 and 17 is provided with aligned pairs of concavities, as at 20 and 21. It will be noted that leaflets 16 and 17 are substantially identical, one to another, with each being the mirror image of the other. Concavities 20 and 21 are designed along the edge surfaces to receive and capture pivot areas 23 and 24 therewithin, as illustrated in Figures 2 and 5. Specifically, the utilization of the spherical projections or pivot areas 23 and 24 reduces the area impeding blood flow, thereby providing an increase in the effective cross-sectional area available for flow through the body 11. The dimensional tolerances between the concavities 20 and 21 and the pivot areas 23 and 24 are such that little, if any, transitional movement of the leaflets 16 and 17 is permitted. The dimensional tolerances are typically such that a normal gap of about 0.0254mm (0.001 inch) exists between the outer surfaces of the pivot areas and the inner surfaces of the concavities, with this dimension being sufficient to accommodate free movement or flow without creation or generation of significant areas of stasis.

With continued attention being directed to Figures 2-4 of the drawings, a plurality of stop means are provided which extend inwardly of the oppositely disposed flat surfaces 26 and 27 (Figure 1). These flat surfaces as at 26 and 27 are parallelly disposed inwardly facing surfaces which are formed generally along chordal planes of the interior surface of the annular body. These flat surfaces are tapered and/or flared outwardly in the direction of flow and toward the base of the body member 11 so as to become coincidental with and merge into the bottom end 28 (Figure 2) of annular body member 11. The outwardly extending flared area in the flow direction and adjacent the outflow end is illustrated at 29 in Figure 3. It has been found that this design arrangement improves flow and reduces stasis, and also reduces any tendency toward the creation of turbulent flow during the occurrence of normal blood flow and normal functioning of the heart valve prosthesis. Stop means such as shown at 30, 31 and 32 extend inwardly of the flat surfaces 26 and 27. The stop means are provided with relatively elongated abutment surfaces so as to control the extent of pivotal motion of each of the occluder means (leaflets) 16 and 17 so as to achieve opening and closing of the valve. The stop means are, as indicated, arranged laterally of the pivot areas, and thus achieve the function of limiting pivotal motion in opening and closing of the leaflets 16 and 17. Surfaces which contribute to limitation of opening of the leaflets are found at 34, 35, 36, 37, 40 and 41. Inasmuch as the forces being applied to the components of the valve during the periods of blood flow while the leaflets 16 and 17 are open are minimal, there is no compelling need for the creation of unusually elongated linear areas for further reduction of mechanical forces and stresses. In other words, blood damage is more likely to occur during that portion of the cycle when the leaflets of the heart valve prosthesis are closed. Furthermore, it will be noted that the stop means 34-37 inclusive provide for a substantial flow area through the body member 11, and in the direction and along the line of the arrow 14 (Figure 2).

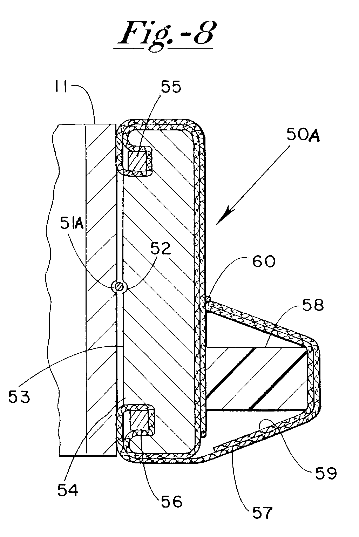

As indicated in Figures 1-6 of the drawings, retaining lock wire accommodating groove 45 is provided on the outer surface of annular body 11. With particular attention now being directed to Figure 8 of the drawings, the sewing cuff assembly generally designated 50A is shown mounted and secured in place on the outer circumferential surface of annular body member 11. This assembly comprises a lock wire 51A adapted to be received within groove 45 formed in annular body 11, as well as within groove 52 formed along the inner circumferential surface 53 of stiffening ring 54. A pair of lock rings 55 and 56 are provided for engaging and appropriately securing fabric 57 in place on assembly 50A. A filler ring 58 is provided within the confines of loop zone 59, with a suture being formed as at 60 to retain loop area 59 of fabric 57 in place on the overall assembly 50A. Typically, stiffening ring 54 and lock rings 55 and 56 are fabricated from a material such as titanium, with lock wire 51A being formed of a suitable blood-compatible, titanium-compatible metal. Fabric 57 may typically be prepared from a double velour material which is commercially available. Filler 58 may conveniently be fabricated from a suitably inert material such as polytetrafluoroethylene which is also commercially available. Stiffening ring 54 has a longitudinal groove formed medially thereof in order to receive an outwardly bent portion of lock wire 51A therethrough, thus serving to retain assembly 50A in tact and in place on the outer surface of annular member 11.

Additionally, it should be pointed out that sewing cuff assembly 50A retains valve assembly 10 in place by frictional engagement between the inner surface of fabric 57 and the outer surface of annular body member 11. When placing the valve assembly in place within the patient's system, sewing cuff 50A is secured in place, and the valve prosthesis 10 may then be rotated to a desirable orientation with respect to its implanted location. In other words, with this configuration, it is, therefore, possible for the implanting surgeon to suture the conventional tubular fabric or sewing cuff in place and thereafter rotatably orient the valve to its desired leaflet opening position after the tubular fabric has been appropriately secured in place.

When the valve leaflets are in their closed disposition, the leaflets 16 and 17 rest upon the abutment surfaces 38, 39, 40 and 41. Additionally, the outer tip portions of the leaflets 16 and 17, as at 42 and 43, are arranged in contact with the interior surface 12 of annular body member 11, as illustrated in Figure 3 at 44 and 45. The straight-line edges of leaflets 16 and 17, as at 47 and 48 respectively are in abutting contact when the leaflets are closed. As indicated hereinabove, there is very little transitional movement of the leaflets 16 and 17 during normal functioning of the valve, with this motion being sufficient and adequate to contribute to a reduction in the mechanical forces being exerted upon mating and contact surfaces of the components within the valve. Specifically, there is only a relatively small amount of rubbing contact created between the rounded surfaces of the leaflets and the inner surface of the annular body 11, thus further contributing to a reduction in the forces exerted upon the components of the blood.

Attention is now directed to Figures 2-4 of the drawings, where the profiles of the individual abutment surfaces and stop means are illustrated. As indicated, the profile of the central stop, as at 30, includes an angularly inwardly projecting portion 30 together with an outwardly flared portion 29. The arrangement of stop means 31 and 32 are also illustrated, with the surfaces 50 and 51 being arranged angularly to the direction of flow of the blood. In this connection, the angle of surfaces 50 and 51 relative to the vertical flow axis is 45 degrees. However, it is noted that this angle may be increased to approximately 60 degrees while preserving the uniformity of flow through the body of the prosthesis.

Turning now to the operation of the heart valve prosthesis 10, upon the occurrence of the natural pumping action of the heart, when the inflow pressure exceeds the outflow pressure, thus causing flow of blood along the line and in the direction of the arrows 13, 14, and 15, leaflets 16 and 17 move or pivot to the open disposition. During the pressure reversal portion of the normal cycle, the leaflets 16 and 17 pivot to their closed disposition as illustrated in Figure 3. Normal heart rhythm which is approximately 72 beats per minute for a human at rest, increases as a result of exercise or the like. In the open disposition, the leaflets are held at an angular disposition which is modestly less than parallel to the flow direction. Specifically, this angle of deflection from the axis of flow (the valve axis) is between about 4 degrees and 6 degrees, and preferably at about 5 degrees. For normal operation, a full 60 degree rotational skirt of motion is provided for the individual leaflets, thereby providing an angle of closure for the individual leaflets at about 25 degrees from a plane normal to the flow direction or annular valve body axis. Thus, a substantial number of cycles of the heart valve prosthesis is expected, and the components of the valve may be made from any suitable material that wall resist wear when subjected to the virtually countless opening and closing movements of the occluder members of the valve. Pyrolytic carbon has been found to be a desirable material of construction for devices of this type. Furthermore, pyrolytic carbon has been found to be highly compatible with blood and its numerous components, is non-thrombogenic, without having presented problems from the standpoint of creation or generation of clotting activity.

## Claims

1. Heart valve prosthesis comprising a generally annular body (11) having an interior surface (12) defining a central passageway for blood flow therethrough; occluder means (16,17) supported on said annular body for alternately blocking and then allowing the flow of blood through said passageway in a predetermined direction (13,14,15), said occluder means having generally planar upstream and downstream major surfaces (18,19) and being formed with aligned concavities (20,21) at opposed locations along the periphery thereof; a pair of opposed flat surfaces (26,27) with mutually parallel, inwardly facing surfaces formed along the interior surface of said annular body; oppositely disposed pairs of segments (23,24) extending inwardly from each of said flat surfaces of said annular body to form pivot areas engaged within said concavities (20,21) with the axes of said segments being arranged along and generally coincidental with the axis of said concavities; a respective stop means (29,30,31,32) extending radially inwardly of each flat surface and including first, second and third abutment pad means, with each associated segment being positioned between the first abutment pad means (29,30) and a respective one of the second and third abutment pad means (31,32), each stop means cooperating with said occluder means and respective pivot areas to limit the extent of angular pivotal movement in the opening and closing of said occluder means, each first abutment pad means (29,30) having a pair (34,35) of abutment surfaces arranged along the edge surfaces thereof and disposed between the associated pivot areas to contact the downstream surface (19) of individual ones of said occluder means to limit the extent of opening thereof, each abutment surface (34,35) having a generally rectangular configuration; and each of the second and third abutment pad means (31,32) having an abutment surface (36,37,40,41) including a first portion (36,37) for contacting the upstream side (18) of an individual one of said occluder means in the open position thereof, and a second portion (40,41) projecting outwardly from the respective pivot areas for contacting the upstream surface (18) of said individual one of said occluder means upon closure thereof,
characterised in that each segment (23,24) is spherical and extends convexly inwardly from the respective flat surface (26,27), each first abutment pad means (29,30) has an upstream portion (30) with an inner surface angled inwardly towards the flow axis in the downstream direction and each of the second and third abutment pad means (31,32) includes an upstream end surface (50,51) angled inwardly towards the flow axis, in the downstream direction.

2. The heart valve prosthesis as defined in Claim 1 being characterised in that each surface of said pair of abutment surfaces (34,35) tapers outwardly away from the flow axis in the downstream direction at an angle of about 5 degrees to the flow axis.

3. The heart valve prosthesis as defined in Claim 1 or 2 being characterised in that said oppositely disposed pairs of spherical segments (23,24) are substantially hemispherical in configuration.

4. The heart valve prosthesis as defined in any preceding claim being characterised in that each of said upstream end surfaces (50,51) intersects the axis of flow at an angle ranging from about 45 degrees to 60 degrees.

5. The heart valve prosthesis as defined in Claim 4 being characterised in that said angle of intersection is about 45 degrees.

6. The heart valve prosthesis as defined in Claim 1 being characterised in that said occluder means (16,17) undergo a rotational skirt of motion between open and closed dispositions of about 60 degrees.

7. The heart valve prosthesis of any preceding claim, wherein each first abutment pad means (29,30) has a downstream portion (29) extending radially outwardly away from the flow axis in the downstream direction.

## Patentansprüche

1. Herzklappenprothese, welche aufweist: einen im allgemeinen ringförmigen Körper (11) mit einer Innenfläche (12), welche einen zentralen Durchtritt für die Blutströmung begrenzt; eine Verschlußeinrichtung (16, 17), die am ringförmigen Körper gehaltert ist, um die Blutströmung durch den Durchtritt in einer vorbestimmten Richtung (13, 14, 15) alternierend zu blockieren und anschließend freizugeben, wobei die Verschlußeinrichtung im allgemeinen ebene stromaufwärts- und stromabwärtsgelegene größere Oberflächen (18, 19) aufweist und mit ausgerichteten Rundhöhlungen (20, 21) an gegenüberliegenden Stellen längs ihres Umfangs ausgebildet ist; ein Paar gegenüberliegende ebene Flächen (26, 27) mit gegenseitig parallelen, nach innen aufeinanderzu gerichteten Oberflächen, die längs der Innenfläche des ringförmigen Körpers ausgebildet sind; gegenüberliegend angeordnete Paare von Segmenten (23, 24), die sich von jeder ebenen Oberfläche des ringförmigen Körpers nach innen erstrecken, um Schwenkbereiche zu bilden, die innerhalb der Rundhöhlungen (20, 21) in Eingriff sind, wobei die Achsen der Segmente längs und im allgemeinen zusammenfallend mit den Achsen der Rundhöhlungen angeordnet sind; eine entsprechende Stoppeinrichtung (29, 30, 31, 32), die sich von jeder ebenen Fläche radial nach innen erstreckt und erste, zweite und dritte Anschlagauflagemittel aufweist, wobei jedes zugeordnete Segment zwischen dem ersten Anschlagauflagemittel (29, 30) und einem entsprechenden zweiten bzw. dritten Anschlagauflagemittel (31, 32) angeordnet ist, wobei jedes Stoppmittel mit der Verschlußeinrichtung und den entsprechenden Schwenkbereichen zusammenwirkt, um das Ausmaß der Winkelschwenkbewegung beim Öffnen und Schließen der Verschlußeinrichtung ZU begrenzen, wobei das erste Anschlagauflagemittel (29, 30) ein Paar (34, 35) von Anschlagflächen aufweist, die längs seiner Randflächen und zwischen den zugeordneten Schwenkbereichen angeordnet sind, um die stromabwartsgelegene Oberfläche (19) von einzelnen Verschlußmitteln zu kontaktieren, um dessen Öffnungsmaß zu begrenzen, wobei jede Anschlagfläche (34, 35) eine im allgemeinen rechtwinkelige Ausgestaltung hat; und wobei jedes zweite und dritte Anschlagauflagemittel (31, 32) eine Anschlagfläche (36, 37, 40, 41) einschließlich eines ersten Abschnittes (36, 37) aufweist, um die stromaufwärtsgelegene Seite (18) eines einzelnen Verschlußmittels in dessen offener Position zu kontaktieren, und einen zweiten Abschnitt (40, 41), der von den entsprechenden Schwenkbereichen nach außen vorsteht, um die stromaufwartsgelegene Fläche (18) des einzelnen Verschlußmittels auf dessen Verschließen hin zu kontaktieren,
**dadurch gekennzeichnet**, daß jedes Segment (23, 24) kugelförmig ist und sich von der entsprechenden ebenen Fläche (26, 27) aus konvex nach innen erstreckt, daß jedes erste Anschlagauflagemittel (29, 30) einen stromaufwärtsgelegenen Abschnitt (30) mit einer inneren Flache hat, die zur Strömungsachse hin in Richtung stromabwärts nach innen in einem Winkel verläuft, und daß jedes zweite und dritte Anschlagauflagemittel (31, 32) eine stromaufwärtsgelegene Endfläche (50, 51) aufweist, die zur Strömungsachse hin nach innen in Richtung stromabwärts in einem Winkel verläuft.

2. Herzklappenprothese nach Anspruch 1, **durch gekennzeichnet**, daß jede Fläche des Paares von Anschlagflächen (34, 35) nach außen in einem Winkel von etwa 5° zur Strömungsachse weg von der Strömungsachse in Richtung stromabwärts konisch verläuft.

3. Herzklappenprothese nach Anspruch 1 oder 2, **durch gekennzeichnet**, daß die gegenüberliegend angeordneten Paare von kugelförmigen Segmenten (23, 24) im wesentlichen halbkugelförmig ausgebildet sind.

4. Herzklappenprothese nach einem der vorhergehenden Ansprüche, **dadurch gekennzeichnet**, daß jede stromaufwärts gelegene Endfläche (50, 51) die Strömungsachse in einem Winkel zwischen etwa 45° und 60° schneidet.

5. Herzklappenprothese nach Anspruch 4, **durch gekennzeichnet**, daß der Schnittwinkel etwa 45° beträgt.

6. Herzklappenprothese nach Anspruch 1, **durch gekennzeichnet**, daß die Verschlußeinrichtung (16, 17) eine Drehbewegung von etwa 60° zwischen der offenen und geschlossenen Position durchführt.

7. Herzklappenprothese nach einem der vorhergehenden Ansprüche, wobei jedes erste Anschlagauflagemittel (29, 30) einen stromabwärtsgelegenen Abschnitt (29) hat, der sich radial auswärts in Richtung stromabwärts weg von der Strömungsachse erstreckt.

## Revendications

1. Prothèse de valvule cardiaque comprenant un corps globalement annulaire (11) ayant une surface intérieure (12) définissant un passage central pour la circulation sanguine à travers celui-ci ; des moyens d'occlusion (16,17) supportés sur ledit corps annulaire pour alternativement retenir puis laisser passer le flux sanguin à travers ledit passage dans une direction prédéterminée (13,14,15), lesdits moyens d'occlusion ayant des surfaces principales mont et aval globalement planes (18,19) et étant formés de concavités alignées (20,21) à des emplacements opposés le long de leur périphérie ; une paire de surfaces planes opposées (26,27) avec des surfaces orientées vers l'intérieur, mutuellement parallèles, formées le long de la surface intérieure dudit corps annulaire ; des paires de segments (23,24) disPosées à l'opposé l'une de l'autre en saillie vers l'intérieur depuis chacune desdites surfaces planes dudit corps annulaire pour former des zones de pivot en prise dans lesdites concavités (20,21), les axes desdits segments étant agencés le long de l'axe desdites concavités et coïncidant globalement avec celui-ci ; des moyens de butée respectifs (29,30,31,32) en saillie radialement vers l'intérieur de chaque surface plane et comprenant des premier, deuxième et troisième moyens formant tampon de butée, chaque segment associé étant positionné entre les premiers moyens formant tampon de butée (29,30) et un des moyens, respectif, des deuxième et troisième moyens formant tampon de butée (31,32), chacun des moyens de butée coopérant avec lesdits moyens d'occlusion et les zones de pivot respectives pour limiter l'étendue du mouvement pivotant angulaire lors de l'ouverture et de la fermeture desdits moyens d'occlusion, chacun des premiers moyens formant tampon de butée (29,30) ayant une paire (34,35) de surfaces de butée agencée le long des surfaces de bord de ceux-ci et disposée entre les zones de pivot associées pour venir en contact avec la surface aval (19) de moyens individuels parmi lesdits moyens d'occlusion pour limiter l'étendue d'ouverture de ceux-ci, chaque surface de butée (34,35) ayant une configuration globalement rectangulaire ; et chacun des deuxième et troisième moyens formant tampon de butée (31,32) ayant une surface de butée (36,37,40,41) comprenant une première partie (36,37) pour venir en contact avec le côté amont (18) de l'un desdits moyens d'occlusion, individuel, dans la position ouverte de ceux-ci, et une deuxième partie (40,41) en saillie vers l'extérieur depuis les zones de pivot respectives pour venir en contact avec la surface amont (18) dudit élément individuel desdits moyens d'occlusion lors de la fermeture de ceux-ci,
caractérisée en ce que chaque segment (23,34) est sphérique et fait saillie de manière convexe vers l'intérieur de la surface plane respective (26,27), en ce que chacun des premiers moyens formant tampon de butée (29,30) comporte une partie amont (30) avec une surface intérieure formant un angle vers l'intérieur en direction de l'axe de circulation dans la direction aval, et en ce que chacun desdits deuxième et troisième moyens formant tampon de butée (31,32) comprend une surface d'extrémité amont (50,51) formant un angle vers l'intérieur en direction de l'axe de circulation, dans la direction aval.

2. Prothèse de valvule cardiaque selon la revendication 1, caractérisée en ce que chaque surface de ladite paire de surfaces de butée (34,35) s'effile vers l'extérieur en partant de l'axe de circulation dans la direction aval à un angle d'environ 5 degrés par rapport à l'axe de circulation.

3. Prothèse de valvule cardiaque selon la revendication 1 ou 2, caracterisée en ce que lesdites paires disposées à l'opposé l'une de l'autre des segments sphériques (23,24) sont de configuration sensiblement hémisphérique.

4. Prothèse de valvule cardiaque selon l'une quelconque des revendications précédentes, caractérisée en ce que chacune desdites surfaces d'extrémité amont (50,51) coupe l'axe de circulation à un angle compris entre 45 degrés et 60 degrés.

5. Prothèse de valvule cardiaque selon la revendication 4, caractérisée en ce que ledit angle d'intersection est d'environ 45 degrés.

6. Prothèse de valvule cardiaque selon la revendication 1, caractérisée en ce que lesdits moyens d'occlusion (16,17) sont soumis à un arc de mouvement de rotation d'environ 60 degrés entre les situations ouverte et fermée.

7. Prothèse de valvule cardiaque selon l'une quelconque des revendications précédentes, dans laquelle chacun des premiers moyens formant tampon de butée (29,30) comporte une partie aval (29) en saillie radialement vers l'extérieur en partant de l'axe de circulation dans la direction aval.
